(19) Europäisches Patentamt
European Patent Office
Office européen des brevets

(11) Veröffentlichungsnummer: **0 001 803**
A1

(12) **EUROPÄISCHE PATENTANMELDUNG**

(21) Anmeldenummer: **78101219.0**

(22) Anmeldetag: **25.10.78**

(51) Int. Cl.$^2$: **C 08 F 20/34**
C 08 F 20/60, C 08 L 101/00
//C07D211/46, C07D211/58,
C07D221/20

(30) Priorität: **28.10.77 DE 2748362**

(43) Veröffentlichungstag der Anmeldung:
**16.05.79 Patentblatt 79/10**

(84) Benannte Vertragsstaaten:
**BE CH DE FR GB LU NL**

(71) Anmelder: **HOECHST AKTIENGESELLSCHAFT**
**Postfach 80 03 20**
**D-6230 Frankfurt/Main 80(DE)**

(72) Erfinder: **Wiezer, Hartmut, Dr.**
**Hans-Fischer-Strasse 6**
**D-8906 Gersthofen(DE)**

(72) Erfinder: **Pfahler, Gerhard, Dr.**
**Karlsbader Strasse 27**
**D-8900 Augsburg(DE)**

(72) Erfinder: **Mayer, Norbert, Dr.**
**Ziegelgrundweg 17**
**D-8901 Gablingen(DE)**

(72) Erfinder: **Knorr, Harald, Dr.**
**Hans-Fischer-Strasse 6**
**D-8906 Gersthofen(DE)**

(54) **Polymere substituierter Piperidine, ihre Herstellung und Verwendung.**

(57) Die Erfindung betrifft neue Homo- und Copolymerisate von 4-Acryloyloxy- oder 4-Acryloylamido-2,2-Dimethyl-6,6-Dialkylpiperidinen bzw. den entsprechenden Methacryloyl-Verbindungen sowie deren Verwendung als Licht- und Wärmestabilisatoren für Thermoplaste, Lackbindemittel und polymere Überzugsmassen.

**EP 0 001 803 A1**

HOECHST AKTIENGESELLSCHAFT HOE 77/F 816 /W

Polymere substituierter Piperidine, ihre Herstellung und Verwendung

Die Erfindung betrifft neue Polymerisate, ein Verfahren zu ihrer Herstellung und ihre Verwendung als Lichtschutzmittel für synthetische Polymere.

Die neuen Polymerisate sind Homo- und Copolymere von substituierten Piperidinen der allgemeinen Formel (I)

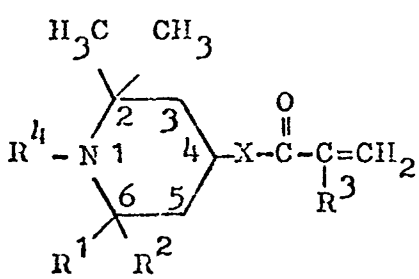

(I)

in welcher

$R^1$ und $R^2$ für gleiche oder verschiedene Alkyl- oder Isoalkylreste mit 1 bis 12, vorzugsweise 1 bis 6 C-Atomen und insbesondere Methylgruppen, oder für einen über das Kohlenstoffatom 6 des Piperidinringes gebildeten, gegebenenfalls methylsubstituierten Cycloalkanring mit 5 bis 12, vorzugsweise 5 oder 6 C-Atomen, und insbesondere einen 2,2,6,6-Tetramethylpiperidinring, stehen,

$R^3$ hat die Bedeutung von Wasserstoff oder Methyl,

$R^4$ ist Wasserstoff oder ein Alkylrest mit 1 bis 18, vorzugsweise 1 bis 4 C-Atomen und

X soll Sauerstoff oder eine $>NR^5$-Gruppe mit $R^5$ = Wasserstoff oder einem $C_1$- bis $C_{12}$-Alkylrest, vorzugsweise Wasserstoff oder einem $C_1$- bis $C_4$-Alkylrest sein.

Bei den Copolymeren kann ein Teil oder die gesamte Menge der Cokomponente durch ein Vinylmonomeres der allgemeinen Formeln

$$H_2C=\overset{R^6}{\overset{|}{C}}COOR^7 \quad (II), \qquad H_2C=\overset{R^6}{\overset{|}{C}}CON\langle{}^{R^8}_{R^9} \quad (III),$$

$$H_2C=\overset{R^{10}}{\overset{|}{C}}CH=CH_2 \quad (IV) \text{ oder } \quad H_2C=\overset{R^6}{\overset{|}{C}}-R^{11} \quad (V)$$

ersetzt sein, wobei in diesen Formeln

$R^6$ Wasserstoff oder eine Methylgruppe ist,

$R^7$ einen Alkyl- oder Hydroxyalkylrest mit 1 bis 18, vorzugsweise 1 bis 6 C-Atomen und insbesondere Methyl bedeutet,

$R^8$ und $R^9$ bevorzugt gleich, gegebenenfalls aber auch verschieden sind und die Bedeutung von Wasserstoff, einem Alkylrest mit 1 bis 18, vorzugsweise 1 bis 6 C-Atomen, insbesondere die Bedeutung von Wasserstoff haben,

$R^{10}$ Wasserstoff, Methyl oder Chlor ist und

$R^{11}$ für eine Hydroxylgruppe, Chlor, eine O-Acetylgruppe, eine Acetylgruppe, eine Nitrilgruppe oder einen Phenylrest, vorzugsweise eine O-Acetylgruppe oder einen Phenylrest steht.

Es war überraschend und nicht zu erwarten, daß sich die

stickstoffhaltigen Ausgangsmonomeren der Struktur (I) überhaupt polymerisieren oder copolymerisieren lassen, nachdem aus der DT-OS 2 113 246 bekannt ist, daß Verbindungen, die den Monomeren nach (I) recht ähnlich sind, eine Polymerisationsinhibitorwirkung für Vinylmonomere der Struktur (II), (III), (IV) und (V) besitzen. Man mußte vielmehr annehmen, daß die Piperidinmonomeren der Formel (I) nicht oder nur äußerst unbefriedigend polymerisierbar seien. Daß sich diese Verbindungen dennoch ausgezeichnet homopolymerisieren lassen und außerdem als Monomere für die Copolymerisation mit Vinylmonomeren der Formeln (II), (III), (IV) und (V) hervorragend geeignet sind, konnte somit keineswegs vorhergesehen werden.

Nicht vorhersehbar war schließlich auch die Eignung der neuen Verbindungen als Kunststoffstabilisatoren, da befürchtet werden mußte, daß polymerartige Stabilisatoren mit strukturell andersartigen synthetischen Polymeren nicht genügend verträglich sind, um hinreichend wirksam sein zu können.

Die erfindungsgemäßen Homo- und Copolymeren lassen sich nach bekannten Verfahren für die Polymerisation von Acrylsäure- und Methacrylsäurederivaten herstellen (vgl. z.B. Houben-Weyl Bd. 14/1, S. 1026 - 1079). Genannt seien beispielsweise die Lösungs-, Emulsions- und Dispersionspolymerisation unter Verwendung üblicher Katalysatoren, vorzugsweise $\alpha,\alpha'$-Azodiisobutyronitril und Peroxidisulfate, wobei Polymere mit Molekulargewichten von $10^3$ bis $10^7$, vorzugsweise $1 \times 10^3$ bis $10^5$ und insbesondere $1 \times 10^3$ bis $5 \times 10^4$ gebildet werden.

Dem Umfang der Erfindung entsprechend, wird entweder ein Monomeres der Formel (I) polymerisiert, wobei ein Piperidylhomopolymerisat erhalten wird, oder man polymerisiert eine Mischung aus mindestens zwei verschiedenen Monomeren der Formel (I), was zu Piperidylcopolymerisaten führt. Es ist ferner möglich, Copolymerisate herzustellen, die aus mindestens einem Monomeren der Formel (I) und

mindestens einem Vinylmonomeren der Formeln (II), (III), (IV) oder (V) bestehen.

In den Copolymerisaten kann das Monomerenverhältnis in weiten Grenzen schwanken, es ist jedoch dafür Sorge zu tragen, daß das Piperidylmonomere (I) in einer so großen Menge einpolymerisiert ist, daß das resultierende Copolymerisat die gewünschte Eigenschaft, nämlich, in Plasten eingearbeitet, diese gegen den schädigenden Einfluß von Licht zu schützen, noch in ausreichendem Maße besitzt, bei Einsatz von in der Praxis üblichen Stabilisatormengen von 0,01 bis 5,0 Gewichtsteilen auf 100 Gewichtsteile Plast. Bei unter diesem Aspekt hergestellten Copolymeren liegt - statistisch gesehen - das Verhältnis von Monomerkomponente (I) zu dem oder zu den Vinylmonomeren der Struktur (II), (III), (IV) oder (V) bei etwa 1 : 0,5 bis 1 : 50, vorzugsweise 1 : 0,5 bis 1 : 20. Als besonders geeignete Stabilisatoren haben sich Produkte vom Molverhältnis 1 : 0,5 bis 1 : 5 erwiesen. Es sei schließlich die Möglichkeit aufgezeigt, die Piperidylmonomeren gemäß Formel (I) bei der Herstellung von Homo- und Copolymeren der Vinylverbindungen entsprechend den Formeln (II), (III), (IV) und (V) d i r e k t in der zu deren Stabilisierung ausreichenden Menge einzupolymerisieren. Das Molverhältnis liegt in diesem Falle im allgemeinen bei 1 : 20 000 bis 1 : 200.

Bei der Polymerisation wird im einzelnen so vorgegangen, daß man im Falle der Lösungspolymerisation das Monomere oder das Monomerengemisch in einem organischen Lösungsmittel, wie z. B. Toluol, Xylol oder auch in Wasser, bei stark polaren Monomeren, unter Zusatz von 0,01 bis 15 Gewichtsprozent, vorzugsweise 2 bis 15 Gewichtsprozent eines üblichen Radikalstarters, vorzugsweise $\alpha,\alpha'$-Azodiisobutyronitril oder Peroxodisulfat, bei Temperaturen von 50 bis 180, vorzugsweise 80 bis 150 und insbesondere 90 bis 120°C, 3 bis 30, vorzugsweise 10 bis 20 Stunden polymerisiert. Bedient man sich des Verfahrens der Emulsionspolymerisation, so wird das Monomere oder das Monomerengemisch in Wasser, welches 2 bis 6 Gewichtsprozent eines Emulgators enthält, in Gegenwart von 0,01 bis 5, vorzugsweise 0,01 bis 2 Gewichtsprozent des Katalysators bei 70 bis 100°C polymerisiert.

Die neuen Polymeren und Copolymeren werden aus den Lösungen bzw. Emulsionen durch Ausfällen mit geeigneten Lösungsmitteln wie z.B. Alkoholen oder aliphatischen Kohlenwasserstoffen, als Feststoffe bzw. weiche Massen gewonnen. Falls erforderlich, können die Polymerisate durch Umfällen gereinigt werden. Selbstverständlich erübrigt sich das Ausfällen, wenn man nach dem Verfahren der Dispersionspolymerisation arbeitet.

Die Monomeren der Formel (I) sind z.B. aus der DT-OS 2 040 983 bekannt und entsprechend den dort gemachten Angaben zugänglich. Genannt seien beispielsweise 4-Acryloyloxy-2.2.6.6-tetramethylpiperidin, 4-Methacryloyloxy-2.2.6.6-tetramethylpiperidin, 4-Acrylamido-2.2.6.6-tetramethylpiperidin und 4-Methacrylamido-2.2.6.6-tetramethylpiperidin.

Monomere der Formel (II) sind beispielsweise Acrylsäuremethylester, Acrylsäureäthylester, Acrylsäurebutylester, Acrylsäure-2-äthylhexylester, Methacrylsäuremethylester, Methacrylsäurebutylester, Methacrylsäuredecylester und Methacrylsäurehydroxypropylester.
Zur Gruppe der Monomeren der Formel (III) gehören z.B. Acrylsäureamid, Acrylsäuremethylamid, Acrylsäureoctadecylamid, Acrylsäuredibutylamid, Methacrylsäureamid, Methacrylsäureoctadecylamid und Methacrylsäurediäthylamid.

Unter Monomeren der Formel (IV) sind z.B. zu verstehen Isopren und Butadien.

Monomere, die durch die Formel (V) charakterisiert werden, sind z.B. Vinylacetat, Acrylnitril und Styrol.

Die neuen piperidinhaltigen Polymeren, die eine sehr geringe Flüchtigkeit besitzen, eignen sich in hervorragender Weise zum Stabilisieren von synthetischen Polymeren gegen den Abbau durch Licht und Wärme. Auch zum Stabilisieren von Lackbindemitteln und polymeren Überzugsmassen sind sie brauchbar.

Unter synthetischen Polymeren werden verstanden: halogenfreie und halogenhaltige Homo- und Copolymere, im einzelnen Homopolymerisate von Olefinen, Dienen und Styrol, wie z.B. Polyäthylen niedriger und hoher Dichte, Polypropylen, Polystyrol, Polybutadien und Polyisopren, Copolymere von Olefinen, Dienen und Styrol miteinander oder mit anderen olefinisch ungesättigten Monomeren, wie Äthylen-Propylen-Copolymere, Äthylen-Buten-Copolymere, Styrol-Butadien-Copolymere, Äthylen-Vinylacetat-Copolymere und Acrylnitril-Butadien-Styrol-Copolymere, Homopolymere von Vinylchlorid und Vinylidenchlorid und Copolymere dieser Monomeren untereinander und mit anderen olefinisch ungesättigten Monomeren. Des weiteren sollen auch Polyurethane, Polyacetale, Polyester, Polyamide, Polyacrylate und Epoxyharze eingeschlossen sein. Bevorzugt sind Poly-$\alpha$-Olefine wie Polyäthylene und insbesondere Polypropylene, sowie die Polymeren des Vinylchlorids.

Die neuen stabilisierenden Polymeren werden nach allgemein üblichen Methoden in die Polymermassen eingearbeitet. Alternativ kann man auch eine Lösung, Suspension oder Emulsion des Stabilisators mit dem Polymeren direkt oder mit einer Lösung, Suspension oder Emulsion desselben vermischen und das Lösungsmittel anschließend entfernen.

Die Stabilisatoren gemäß der Erfindung sind für sich allein oder im Gemisch mit einem oder mehreren der bei der Kunststoffverarbeitung üblichen Stabilisatoren, wie z.B. Antioxidantien, auf Phenol- und Sulfidbasis, UV-Absorbern und Lichtschutzmitteln, Phosphitstabilisatoren, Metallverbindungen, Epoxystabilisatoren und mehrwertigen Alkoholen einsetzbar. In den zu stabilisierenden Kunststoffmassen können ferner Flammschutzmittel und Pigmente,

Farbstoffe, Antistatika und Füllstoffe, wie z.B. Glasfasern, anwesend sein.

Beispiele für geeignete Antioxidantien sind solche vom Typ der sterisch gehinderten Phenole wie 2,6-Di-tert.-butyl-p-kresol, 2,6-Di-octadecyl-p-kresol, 4,4'-Butyliden-bis-(2,6-di-tert.-butyl-phenol), 4,4'-Thio-bis-(2-tert.-butyl-5-methylphenol), phenolische Triazinverbindungen, Thiodipropionsäureester von Fettalkoholen, Dioctadecylsulfid und -disulfid.

Zu den UV-Absorbern und Lichtschutzmitteln gehören z.B. 2-(2'-Hydroxyphenyl)-benztriazole wie 2-(2'-Hydroxy-5'-methylphenyl)-benztriazol, 2-Hydroxybenzophenone wie 2-Hydroxy-4-octoxy-benzophenon, Stabilisatoren aus der Gruppe der Salizylate wie Octylphenylsalizylat, Nickelchelate, Oxalsäurediamide und sterisch gehinderte Piperidinverbindungen.

Als Phosphite sind Trisnonylphenylphosphit, Trislaurylphosphit oder auch Ester des Pentaerythritphosphits zu nennen.

Unter als Stabilisatoren bekannten Metallverbindungen werden in diesem Zusammenhang verstanden: Calcium-, Barium-, Strontium-, Zink-, Cadmium-, Magnesium-, Aluminium- und Bleiseifen aliphatischer Carbonsäuren oder Oxycarbonsäuren mit etwa 12 bis 32 C-Atomen, Salze der genannten Metalle mit aromatischen Carbonsäuren wie Benzoate oder Salizylate sowie (Alkyl)-Phenolate dieser Metalle, ferner Organozinnverbindungen, wie z.B. Dialkylzinnthioglykolate und Carboxylate.

Bekannte Epoxystabilisatoren sind z.B. epoxidierte höhere Fettsäuren wie epoxidiertes Sojabohnenöl, Tallöl, Leinöl oder epoxidiertes Butyloleat sowie Epoxide langkettiger Olefine.

Mehrwertige Alkohole können beispielsweise Pentaerythrit,

Trimethylolpropan, Sorbit oder Mannit sein, d. h. bevorzugt Alkohole mit 5 oder 6 C-Atomen und 3 bis 6 OH-Gruppen.

Eine wirksame Stabilisatorkombination für Poly-α-Olefine, wie z.B. Hoch-, Mittel- Niederdruckpolymerisate von $C_2$- bis $C_4$-α-Olefinen, insbesondere Polyäthylen und Polypropylen odor von Copolymerisaten derartiger α-Olefine besteht, bezogen auf 100 Gewichtsteile Polymer, beispielsweise aus 0,01 bis 5 Gewichtsteilen einer der erfindungsgemäß zu verwendenden Verbindungen, 0,05 bis 5 Gewichtsteilen eines phenolischen Stabilisators, gegebenenfalls 0,01 bis 5 Gewichtsteilen eines schwefelhaltigen Costabilisators sowie gegebenenfalls 0,01 bis 3 Gewichtsteilen einer basischen oder neutralen Metallseife, wie z.B. Calciumstearat oder Zinkstearat sowie gegebenenfalls 0,1 bis 5 Gewichtsteilen eines Phosphits und gegebenenfalls 0,01 bis 5 Gewichtsteilen eines bekannten UV-Stabilisators aus der Gruppe der Alkoxyhydroxybenzophenone, 4-Hydroxyphenylbenzotriazole, Benzylidenmalonsäuremononitrilester oder der sog. Quencher, wie z.B. Nickelchelate.

Die folgenden Beispiele dienen der näheren Erläuterung der Erfindung.

Beispiel 1:

Poly-4-Methacryloyloxy-2,2,6,6-tetramethylpiperidin

In 50 g Toluol werden 11,2 g 4-Methacryloyloxy-2,2,6,6-tetramethylpiperidin unter Zusatz von 0,6 g (5,35 %) Azodiisobutyronitril (AIBN) 10 Stunden bei 95°C polymerisiert. Die Reaktionsmischung wird sodann in 400 ml Petroläther eingerührt, wobei das gebildete Polymerisat ausfällt, welches man abtrennt und trocknet. Die Substanz schmilzt im Bereich von 140 bis 200°C und besitzt ein mittleres Molgewicht von 5100. Stickstoffgehalt 6,2 Gew.-%.

Beispiele 2 bis 21:

Die Polymeren werden analog Beispiel 1, jedoch unter Verwendung der jeweils angegebenen Monomeren entsprechend den Formeln (I), (II), (III), (IV) und (V), 100 g Toluol als Lösungsmittel und 1,2 g AIBN als Katalysator, hergestellt.

In Tabelle 1 sind die Versuchsdaten zusammengestellt.

Tabelle 1

| Beispiel | Monomeres nach Formel I | II bis V | Eigenschaften des Polymeren: klare Schmelze bei ... °C | Molgewicht | % N |
|---|---|---|---|---|---|
| | und Menge in Gramm | | | | |
| 2 | MAP (3) | MMA (10) | 115 | 1960 | 2,1 |
| 3 | AP (5,7) | MMA (20) | 142 | 2600 | 1,2 |
| 4 | MAP (4,5) | MMA (10) | 140 | 2780 | 2,2 |
| 5 | AP (8,4) | MMA (20) | 135 | 1750 | 1,5 |
| 6 | MAP (9) | MMA (10) | 155 | 2670 | 3,1 |
| 7 | MAP (9) | MA (8,6) | 150 | 4090 | 3,5 |
| 8 | AP (8,4) | MMA (10) | 175 | 3470 | 1,8 |
| 9 | MAP (11,2) | MMA (5) | 120 | 2580 | 4,3 |
| 10 | AP (10,6) | MMA (5) | 110 | 1950 | 4,0 |
| 11 | MAP (4,5) | MMA (20) | 250 | 3470 | 1,5 |
| 12 | AP (4,2) | MMA (20) | 145 | 2600 | 1,1 |
| 13 | MAP (4,5) | MA (17,2) | *) | - | - |
| 14 | AP (4,2) | MA (17,2) | *) | - | - |
| 15 | MAP (6,0) | MA (17,2) | *) | - | - |
| 16 | AP (5,7) | MA (17,2) | *) | - | - |
| 17 | AP (8,4) | MA (17,2) | *) | - | - |
| 18 | MAP (4,5) | MA (8,6) | *) | - | - |
| 19 | AP (8,4) | MA (8,6) | *) | - | - |
| 20 | MAP (11,2) | MA (4,3) | *) | - | - |
| 21**) | AP (11,2) | MMA (25,0) | 170 | 2510 | 1,5 |

*) gummiartiges Polymerisat **) halbe Menge Katalysator

MAP = 4-Methacryloyloxy-2,2,6,6-tetramethylpiperidin

AP = 4-Acryloyloxy-2,2,6,6-tetramethylpiperidin

MMA = Methylmethacrylat

MA = Methylacrylat

Beispiele 22 bis 37:

Es wird analog Beispiel 1 gearbeitet unter Einsatz von Monomeren der Formeln (I) bis (V), 50 g Toluol als Lösungsmittel und 0,4 g Azodiisobutyronitril als Katalysator. Tabelle 2 enthält die Versuchsdaten.

Tabelle 2

| Beispiel | Monomeres nach Formel I und Menge in Gramm | Monomeres nach Formel II bis V und Menge in Gramm | Eigenschaften des Polymeren: klare Schmelze bei ... °C | Molgewicht | % N |
|---|---|---|---|---|---|
| 22 | MAP (2,25) | MMA (10) | 135 | 4190 | 1,4 |
| 23 | AP (3,0) | MA (1,2) | *) | - | - |
| 24 | MAP (3,0) | MMA (10,0) | 200 | 4070 | 1,7 |
| 25 | MAP (4,5) | MMA (10,0) | 200 | 3290 | 2,0 |
| 26 | MAP (9,0) | MMA (10,0) | 195 | 2630 | 3,1 |
| 27 | MAP (11,2) | MMA (5,0) | 165 | 2140 | 4,4 |
| 28 | MAP (4,5) | MA (17,2) | *) | - | - |
| 29 | MAP (6,0) | MA (17,2) | *) | - | - |
| 30 | MAP (4,5) | MA (8,6) | *) | - | - |
| 31 | MAP (9,0) | MA (8,6) | *) | - | - |
| 32 | MAP (11,2) | MA (4,3) | 200 | 2490 | 4,9 |
| 33 | MAP (5,7) | MA (8,6)<br>MMA (5,0) | 139 | 4990 | 1,8 |
| 34 | MAP (2,2) | STY (10,4) | 155 | 9720 | 0,8 |
| 35 | MAP (11,2) | VINA(21,5) | 190 | 6790 | 3,5 |
| 36 | MAP (2,2) | MA (8,6)<br>STY (10,4) | 130 | 3920 | 1,4 |
| 37 | MAP (5,6) | AA (7,1)<br>ÄHA (9,2) | 160 | - | 5,3 |

*) gummiartiges Polymerisat

MAP = 4-Methacryloyloxy-2,2,6,6-tetramethylpiperidin
AP = 4-Acryloyloxy-2,2,6,6-tetramethylpiperidin
VINA = Vinylacetat
MMA = Methylmethacrylat
MA = Methylacrylat
AA = Acrylsäureamid
ÄHA = Äthylhexylacrylat
STY = Styrol

Beispiel 38:

Copolymerisation von Methacrylsäuremethylester mit 4-Acryloyloxy-2,2,6,6-tetramethylpiperidin

50 g Wasser 0,35 g Natriumlaurylsulfat und 0,17 g Ammoniumperoxodisulfat werden auf 80°C erhitzt. Dann wird eine Mischung aus 7,0 g 4-Acryloyloxy-2,2,6,6-tetramethylpiperidin und 31,5 g Methacrylsäuremethylester langsam zugetropft. 15 Minuten nach Beendigung der Zugabe fügt man noch 0,35 g Ammoniumperoxodisulfat in 10 ml Wasser zu und rührt 2 Stunden nach. Gegen Ende der Reaktion fällt das Polymere beim Abkühlen aus. Das mittlere Molgewicht beträgt 17 780. Erhitzt man das Produkt, so liegt bei 270°C eine klare Schmelze vor. Stickstoffgehalt 0,5 Gew.%.

Beispiel 39:

Dieses Beispiel zeigt die lichtstabilisierende Wirkung der erfindungsgemäßen Verbindungen beim Einsatz in einem Poly-$\alpha$-Olefin, ermittelt über die Abnahme der Reißdehnung des Thermoplasten durch Lichteinwirkung.

100 Gewichtsteile Polypropylen mit einem Schmelzindex $i_5$ von ca. 6 g/10 min. (bestimmt nach ASTM D 1238-62 T) und einer Dichte von 0,96 wurden mit

0,1 Gew.-Teilen Pentaerythrityl-tetrakis-[3-(3,5)-di-tert.-butyl-4-hydroxyphenyl)-propionat],
0,2 Gew.-Teilen Calciumstearat und
0,1 Gew.-Teilen des zu prüfenden erfindungsgemäßen Stabilisators vermischt.

Um eine möglichst gleichmäßige Verteilung auf dem Polymerkorn zu erreichen, wurden die Stabilisatoren in einem Lösungsmittel gelöst, und die Lösung wurde unter Rühren in das Polypropylenpulver eingetropft, wobei durch gleichzeitige Bestrahlung mit einer IR-Lampe der größte Teil des Lösemittels wieder abdampfte. Nach ca. 20 Minuten wurde das Calciumstearat hinzugegeben und

noch weitere 10 Minuten gemischt. Lösemittelreste wurden durch Trocknen bei 50°C/120 Min. im Trockenschrank entfernt.

Die Prüfmischung wurde auf einer Windsor-Spritzgußmaschine der Type SP 50 bei 250°C zu 60 x 60 x 1 mm-Platten verspritzt. Aus diesen Platten wurden Prüfkörper nach DIN 53 455 Form 3, verkleinert im Maßstab 1:3, ausgestanzt. Die als Vergleichsmuster benötigten Prüfkörper wurden analog, jedoch unter Fortlassen des zu testenden Stabilisators (Versuch a) bzw. unter Verwendung bekannter Lichtstabilisatoren (Versuche b bis e) hergestellt.

Die Prüfung der Lichtbeständigkeit erfolgte in einer Xenotest-450-Apparatur der Firma Original Hanau Quarz-Lampen GmbH mit der Filterkombination 6 IR + 1 UV gemäß DIN 53 387 "Kurzprüfung der Wetterbeständigkeit". Während der Belichtungszeit betrug die Schwarztafeltemperatur 43°C ± 1°C, die relative Luftfeuchtigkeit im Probenraum 70 % ± 1 %. Der Probenraum wurde alle 2 Stunden 5 Minuten lang mit Frischluft gespült. Die Reißdehnung wurde auf einer Zugprüfmaschine der Firma Instron bei einer Abzugsgeschwindigkeit von 5 cm/Min. nach einer definierten Belichtungszeit ermittelt. Die Ergebnisse sind in der nachstehenden Tabelle zusammengestellt.

Der Stabilitätsfaktor ergibt sich aus dem Verhältnis der Bestrahlungszeit des stabilisierten zur Bestrahlungszeit des nicht stabilisierten Probekörpers, wobei jeweils so lange bestrahlt wurde, bis die Reißdehnung auf die Hälfte des Ausgangswertes gefallen ist.

| Versuch | Stabilisator | Stabilitätsfaktor |
|---|---|---|
| a) | ohne | 1 |
| b) | Vergleich 1 [1)] | < 2,5 |
| c) | Vergleich 2 [2)] | < 2,5 |
| d) | Vergleich 3 [3)] | 3,0 |
| e) | Vergleich 4 [4)] | 3,8 |
| f) | nach Beispiel 5 | > 4,0 |
| g) | nach Beispiel 7 | > 4,0 |
| h) | nach Beispiel 26 | > 4,0 |
| i) | nach Beispiel 34 | > 4,0 |

1) = 2-Hydroxy-4-n-octyloxybenzophenon

2) = 2-(2'-Hydroxy-3',5'-di-tert.-butylphenyl)-5-chlorbenztriazol

3) = 2,2,6,6-Tetramethyl-4-hydroxy-iminopiperidin

4) = 2,2,6,6-Tetramethyl-4-hydroxy-4-cyanopiperidin

## Beispiel 40

Dieses Beispiel zeigt den Einsatz eines erfindungsgemäßen Polymerisates als Stabilisator in einem Lack.

a) Herstellung des Polymerisats

Eine Mischung aus 17 g 4-Methacroyloxy-2,2,6,6-tetramethylpiperidin, 10 g Hydroxypropylmethacrylat, 10 g Butylmethacrylat und 10 g Butylarylat wird innerhalb von 4 Stunden zusammen mit 2 g Azo-bis-isobutyronitril in 50 g siedendes Butylacetat eingetropft. Nach insgesamt 12 Stunden ist die Polymerisation abgeschlossen und eine bei 150°C eingedampfte Probe zeigt den theoretischen Festkörpergehalt von 47 Gew.-%. Für den Einsatz wurde eine 64.4 Gew.-%ige Lösung in Butylacetat hergestellt.

b) Durchführung der Prüfung

Jeweils 100 g eines Thermosetting-Acrylat-Klarlacks (TSA) mit einem Feststoffanteil von 40 Gew.-% wurden unstabilisiert, mit bekannten Stabilisatoren stabilisiert sowie mit dem erfindungsgemäßen Copolymerisat nach a) stabilisiert, auf Prüfbleche aufgesprüht, wobei folgendermaßen vorgegangen wurde:
Auf einem in der Automobilindustrie für Farbfolgen üblichen Untergrund (Zinkphosphatierung, Elektrotauchgrund, Füller) wurde eine 2-Schicht-Metallic-Lackierung (Aluminiumbronce-haltig) aufgespritzt, Schichtdicke ca. 15 Micrometer, und hierauf die Klarlackschicht aufgesprüht und 20 Min. bei 140°C eingebrannt. Dicke dieser Schicht ca. 35 Micrometer.

Die Klarlackansätze waren wie folgt zusammengesetzt:

| Ansatz Nr. | Stabilisator I (g) | Stabilisator II (g) | Klarlack (g) |
|---|---|---|---|
| 1 | - | - | 100 |
| 2 | 0,2 1) | 0,2 2) | 100 |
| 3 | 0,8 3) | 0,2 2) | 100 |

1) Stabilisator der Formel

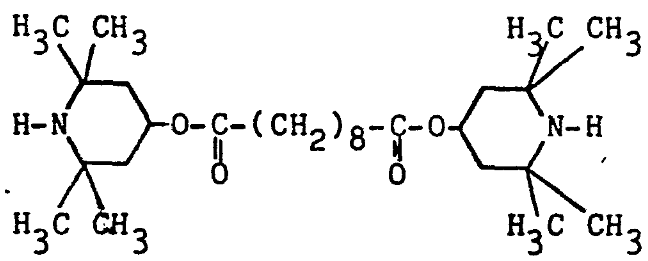

2) Stabilisator der Formel

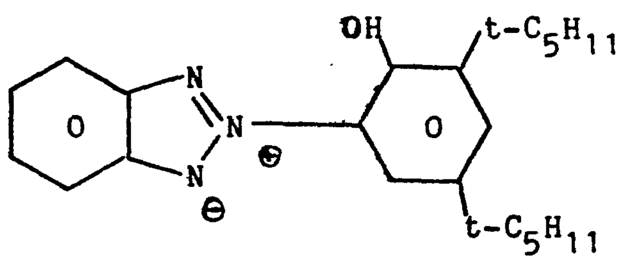

3) Lösung des Produkts nach Beispiel 40a, enthält dieselbe Menge wirksamen Stickstoff wie der Stabilisator I in Ansatz 2.

Die lackierten Bleche wurden der Kurzbewitterung in einem Xenotestgerät 1200 (nach DIN 53 887 "Kurzprüfung der Wetterbeständigkeit") unterworfen. Bewitterungscyclus: 10 Minuten trocken, 5 Minuten beregnen. Schwarztafeltemperatur 74°C am Ende der Trockenperiode. Relative Luftfeuchtigkeit 95 %, Sprühwassertemperatur 45°C, Probenraumtemperatur 45°C.

Die Prüfung is als beendet anzusehen, wenn eine Rißbildung des Lackfilmes beobachtet wird.

Wie aus nachstehender Tabelle ersichtlich, ist der Auftrag mit unstabilisiertem Lack aus Ansatz 1 nach 1300 Stunden rissig geworden, während die Beschichtungen mit den Lacken der Ansätze 2 und 3 bei Abbruch des Versuchs nach 3500 Stunden noch unversehrt waren.

| Lack nach Ansatz Nr. | Zeit bis zur Rißbildung (Stunden) |
|---|---|
| 1 | 1 300 |
| 2 | >3 500 |
| 3 | >3 500 |

Patentansprüche:

1. Homo- und Copolymere substituierter Piperidine der allgemeinen Formel (I)

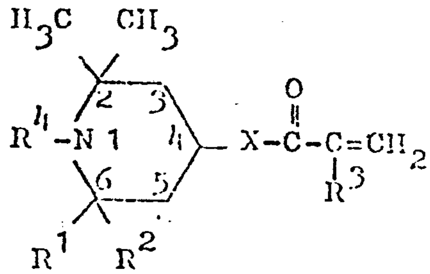

(I)

in der

$R^1$ und $R^2$ für gleiche oder verschiedene Alkyl- oder Isoalkylreste mit 1 bis 12 C-Atomen, oder für einen über das Kohlenstoffatom 6 des Piperidinringes gebildeten, gegebenenfalls methylsubstituierten Cycloalkanring mit 5 bis 12 C-Atomen oder einen 2,2,6,6-Tetramethylpiperidinring, stehen

$R^3$ Wasserstoff oder Methyl bedeutet,

$R^4$ Wasserstoff oder ein Alkylrest mit 1 bis 18 C-Atomen und

X Sauerstoff oder eine $>NR^5$-Gruppe mit $R^5$ = Wasserstoff oder $C_1$- bis $C_{12}$-Alkyl ist,

wobei bei den Copolymeren ein Teil oder die gesamte Menge der Cokomponente durch ein Vinylmonomeres der allgemeinen Formeln

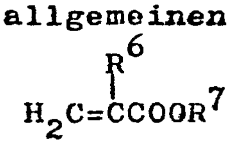

(II),

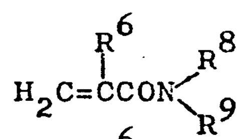

(III),

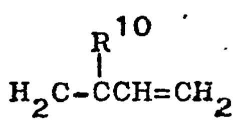

(IV) oder

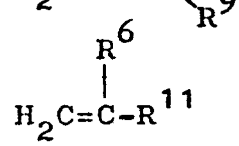

(V),

in denen

$R^6$ Wasserstoff oder Methyl bedeutet,

$R^7$ einen Alkyl-*) mit 1 bis 18 C-Atomen ist,

$R^8$ und $R^9$ gleich oder verschieden sind und die Bedeutung von Wasserstoff oder einem $C_1$- bis $C_{18}$-Alkylrest haben,

$R^{10}$ Wasserstoff, Methyl oder Chlor und

*) oder Hydroxyalkylrest

$R^{11}$ eine Hydroxylgruppe, Chlor, eine O-Acetylgruppe, eine Acetylgruppe, eine Nitrilgruppe oder ein Phenylrest ist,

ersetzt sein kann.

2. Polymere nach Anspruch 1, in denen $R^1$ und $R^2$ Methylgruppen sind und $R^4$ Wasserstoff ist.

3. Verfahren zur Herstellung der Polymerisate aus substituierten Piperidinen gemäß den Ansprüchen 1 und 2, dadurch gekennzeichnet, daß man ein Monomeres der Formel (I) bzw. Mischungen von Monomeren der Formel (I) oder Mischungen aus mindestens einem Monomeren der Formel (I) und mindestens einem Monomeren gemäß den Formeln (II), (III), (IV) oder (V) in einem organischen Lösungsmittel oder im wäßrigen Medium unter Zusatz von 0,01 bis 15 Gew.-% eines Radikalstarters und gegebenenfalls in Anwesenheit von 2 bis 6 Gew.-% eines Emulgators bei Temperaturen von 50 bis 180°C polymerisiert.

4. Verwendung der Polymeren nach einem der Ansprüche 1 und 2 zum Stabilisieren von synthetischen Polymeren.

5. Verwendung nach Anspruch 4, dadurch gekennzeichnet, daß das Polymere ein Polyolefin ist.

6. Verwendung nach Anspruch 4, dadurch gekennzeichnet, daß das Polymere ein halogenhaltiges Polymeres ist.

7. Verwendung nach Anspruch 4, dadurch gekennzeichnet, daß das Polymere ein Polyacrylat oder Polymethacrylat ist.

8. Verwendung nach Anspruch 4, dadurch gekennzeichnet, daß das Polymere ein Homo- oder Copolymeres von Styrol ist.

9. Verwendung der Polymeren nach einem der Ansprüche 1 und 2 zum Stabilisieren von Lackbindemitteln und polymeren Überzugsmassen.

10. Verfahren zum Stabilisieren von synthetischen Polymeren gegen den schädigenden Einfluß von Licht, dadurch gekennzeichnet, daß man dem Polymeren, gegebenenfalls neben bisher bekannten, stabilisierend wirkenden Stoffen, 0,01 bis 5 Gewichtsteile, bezogen auf 100 Gewichtsteile Polymeres, einer Verbindung nach einem der Ansprüche 1 und 2 zusetzt.

11. Gegen UV-Zersetzung stabilisierte, synthetische Polymere, in denen 0,01 bis 5 Gewichtsteile, bezogen auf 100 Gewichtsteile Polymeres, einer Verbindung nach einem der Ansprüche 1 und 2 enthalten sind.

Europäisches Patentamt

# EUROPÄISCHER RECHERCHENBERICHT

Nummer der Anmeldung
EP 78 10 1219

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | DE - A - 2 040 983 (SANKYO)<br>* Patentansprüche 1,2; Seite 2, Zeilen 6-29 *<br>---- | 1-11 |

KLASSIFIKATION DER ANMELDUNG (Int Cl.²)

C 08 F 20/34
20/6
C 08 L 101/00
C 07 D 211/46
211/58
221/20

RECHERCHIERTE SACHGEBIETE (Int. Cl.²)

C 08 F 20/34
20/60
C 08 L 101/00
57/00
C 07 D 211/46
211/58
221/20

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 18-01-1979 | CAUWENBERG |